(19) Europäisches Patentamt
European Patent Office
Office européen des brevets

(11) **EP 4 303 748 A1**

(12) **EUROPEAN PATENT APPLICATION**

(43) Date of publication:
**10.01.2024 Bulletin 2024/02**

(21) Application number: **22183873.3**

(22) Date of filing: **08.07.2022**

(51) International Patent Classification (IPC):
**G06F 21/62** (2013.01)          **G06F 21/64** (2013.01)

(52) Cooperative Patent Classification (CPC):
**G06F 21/6245; G06F 21/64**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Fresenius Medical Care AG & Co. KGaA
61352 Bad Homburg (DE)**

(72) Inventors:
• **Pospiech, Martin
61352 Bad Homburg (DE)**
• **Witt, Waldemar
61352 Bad Homburg (DE)**

(74) Representative: **Hitzelberger, Christoph Michael
Fresenius Medical Care AG & Co. KGaA
Global Intellectual Property
Frankfurter Straße 6-8
66606 Saarland St. Wendel (DE)**

(54) **METHOD AND SYSTEM FOR HEALTHCARE-ACTIVITY TRIGGERED DATA MANAGEMENT**

(57)     The invention relates to a computer-implemented method and a healthcare-data-management system configured to control data transfers. The invention further relates to a computer-implemented method and a healthcare-data-management system configured to retrieve and store a user's interaction data on a first reinforced-protection data base, wherein the interaction data is used to generate an activity gauge of a user to perform or to trigger a performing of a user procedure stored on the first or a second reinforced-protection data base, wherein the user procedure is associated with a data transfer and depends on the activity gauge.

FIG 2

## Description

## Technical Background

The invention relates to a computer-implemented method and a healthcare-data-management system for healthcare-activity triggered data management.

## Introduction

With the advent of distributed ledgers (DL) and the blockchain (BC) technology, a trusted, because transparent, data-storage system was developed, allowing to exchange a variety of digital assets without any central counterparty. Digital assets such as cryptocurrencies or non-fungible tokens (NFTs) have a huge variety of use cases and are expected to have serious impact on current-day finance, payment infrastructures, gaming, or art. One of the most intriguing applications of BC technology is the unique opportunity to create immutable data objects allowing to transparently verify track records, identify ownership, and authenticity of digital assets and real-world assets being linked hereto. Inherently, BC technology allows to keep track of any minted data objects so that ownership or user rights can clearly be determined and assigned.

New technological developments within the BC infrastructure allowed to fully integrate executable code embedded on BCs, which marked a major step forward toward fully decentralized financial infrastructures. For example, the Ethereum BC was one of the first frameworks providing the integration of on-chain executable code. Such smart contracts provide the unique feature to perform autonomous transaction of digital assets or minted data objects forgery-proof secured by BC technology. With smart contracts all counterparties have the guarantee that essential data transactions are indeed performed if the underlying conditions are met.

In the healthcare sector, on the other hand, trusted and secure data transactions as well as tamper-proof documentations are key to comply with regulatory standards and perform both safe and effective medical treatments. However, tamper-proof documentation of classical data systems is usually not guaranteed. Moreover, since patients that receive medical therapy often interrupt, change, or terminate their prescribed treatments or drug dosage due to the additional burdens accompanied with such medical interventions, tamper-proof documentations of treatments and drug intakes become essential and indispensable to guarantee successful therapies. The worsening of the patient's health condition as well as a loss in the quality of life are only two severe consequences for patients due to non-adherence to medical therapies. It is known that incentives such as monetary compensations for adherence can support patients to better adhere to their life-saving treatment and medications.

For any compensation system, however, it is key to provide safe, secure, and reliable data-transfer technology. Moreover, strict local data-protection standards usually require the handling of medical data such as patient data with utmost care. For example, any usage or exploitation of patient data usually requires the patient's consent. Therefore, solutions for the technical problem of providing data systems being secure-by-design and forgery-proof are required and allow patients or other users to take full control of their data especially in situations where patients become unable to express their will anymore, e.g., after leaving a healthcare program or even after death.

## Invention Summary

It is an objective of the invention to solve the technical problems as described above. According to the invention, this objective is achieved by a computer-implemented method for verifiably controlling data transfers according to claim 1, a computer program according to claim 13, a computer-readable medium according to claim 14, and a healthcare-data-management system for verifiably controlling data transfers according to claim 15.

Therefore, a computer-implemented method is proposed, comprising the steps of retrieving interaction data associated with a user from a first reinforced-protection data base. Hereby, the interaction data include data of an interaction between a user and a healthcare system. The method further comprises the steps of generating an activity gauge associated with the user at least partly based on the interaction data, retrieving requirement data from the first and/or a second reinforced-protection data base, wherein requirement data include information related to the performing of a user procedure associated with a data transfer depending on the activity gauge, and performing and/or triggering the performing of the user procedure depending on the activity gauge.

In addition, a computer program is proposed, comprising instructions which, when the program is executed by a computing device, cause the computing device to carry out a method according the invention.

Moreover, a computer-readable medium is proposed, having stored thereon the computer program.

Furthermore, a healthcare-data-management system for verifiably controlling data transfers is proposed, comprising a data acquisition unit configured to acquire interaction data including data of an interaction between a user and a healthcare system. The healthcare-data-management system further comprises communication means configured to exchange the interaction data with a first reinforced-protection data base and/or with a second reinforced-protection data base wherein the healthcare-data-management system is configured to generate an activity gauge at least partly based on the interaction data, retrieve requirement data from the first and/or second reinforced-protection data base, wherein the requirement data include information related to the performing of a user procedure associated with a data transfer depending on the activity gauge, and perform and/or trigger the performing of the user procedure depending on the activity gauge.

The dependent claims comprise preferred embodiments of the invention.

**Description of Figures**

FIG. 1A shows a symbolic depiction of a system configured to execute the method according to an embodiment of the invention.

FIG. 1B schematically depicts a method according to an embodiment of the invention.

FIG. 2 symbolically shows a user procedure according to an embodiment of the invention.

FIG. 3A shows one example of the activity gauge according to an embodiment of the invention.

FIG. 3B shows another example of the activity gauge according to an embodiment of the invention.

FIG. 3C shows another example of the activity gauge according to an embodiment of the invention.

FIG. 3D shows another example of the activity gauge according to an embodiment of the invention.

FIG. 3E shows one example of a multi-dimensional activity gauge according to an embodiment of the invention.

FIG. 4 shows one example of a quantity gauge according to an embodiment auf the invention.

**Detailed Description**

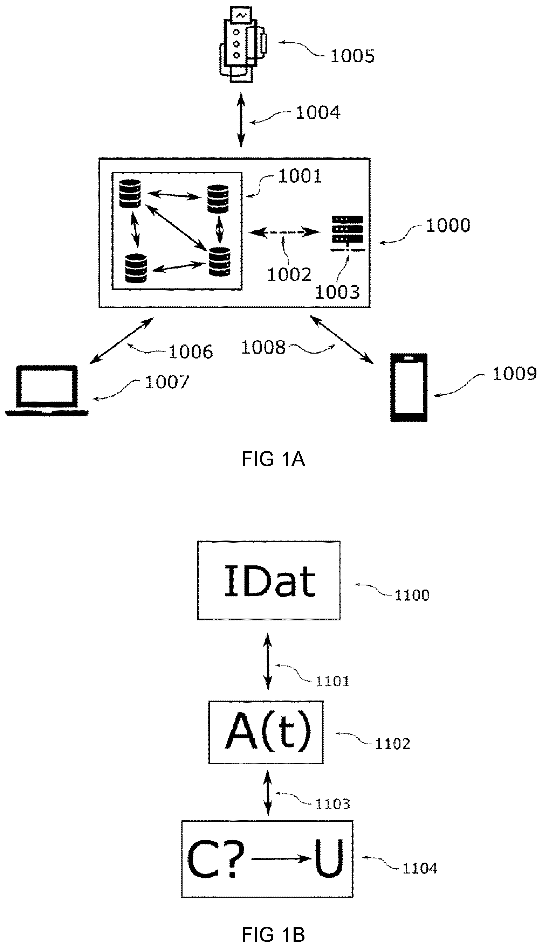

Figure 1A depicts a reinforced-protection data base 1000, a healthcare-data-management system 1005, and external devices 1007, 1009 as part of a system (Fig. 1A), wherein the system is configured to execute the method according to the invention. The reinforced-protection data base 1000 comprises reinforced-protection-data-base nodes, wherein the reinforced-protection-data-base nodes include a DL 1001 and a secured central server 1003, wherein the DL 1001 and the secured central sever 1003 can exchange data 1002 via bidirectional communication means. Reinforced-protection-data-base nodes can include any type of server or computing unit configured to exchange and store data and be configured to set up the reinforced-protection data base 1000. Moreover, the reinforced-protection data base 1000, the healthcare-data-management system 1005, and the external devices 1007, 1009 comprise unidirectional or bidirectional communication means configured to exchange data 1004, 1006, 1008 with the reinforced-protection data base 1000.

The reinforced-protection data base 1000 can be any data base including tamper-proof technologies for preventing unauthorized data access or data manipulation. Such a reinforced-protection data base can include more than a single security technology to provide multiple layers of state-of-the-art security measures to protect sensitive data. The multiple layers of security measures can comprise any of software measures and/or hardware measures for providing data integrity and data security. For example, software measures can include any state-of-the-art user authorization, user identification, ledger-type data architecture, and/or zero-trust software architecture. Hardware measures can include any authorization/identification tokens, storage devices, or computing devices having reinforced security measures to protect the stored, processed, and/or transferred sensitive data.

The healthcare-data-management system 1005 and the external devices 1007, 1009 can also exchange data between each other, e.g., to improve treatments with healthcare devices which can be comprised in the healthcare-data-management system by correlating user data collected from data acquisition units like sensors integrated in the external device or any healthcare devices with data from the respective treatment system, e.g., to control a treatment regimen wherein the collected data are exchanged with the reinforced-protection data base 1000. Such communication means can include any communication technologies such as Ethernet, 5G, Wi-Fi, radio, or fiber-optic communication.

Healthcare-data-management system 1005 can include communication means and/or can communicate via communication means with healthcare systems such as treatment systems, e.g., dialysis systems, infusion systems, radiation-treatment systems and the like. Healthcare systems can also include or can communicate with examination systems, e.g., computer or magnetic-resonance tomography, or computer systems configured to manage and store electronic medical records, e.g., Electronic-Health Record (EHR) systems, or computer systems configured to generate, exchange, and manage medical prescriptions. Healthcare-data-management system 1005 or any healthcare system can also comprise computing units for managing patients, hospitals, clinicians, or health data. The healthcare-data-management system 1005 or any healthcare system can further comprise a data acquisition unit, e.g., a sensor for measuring patient vitals like blood pressure, wherein the data acquisition unit is configured to acquire interaction data of a user, wherein the interaction data include data of an interaction between a user and a healthcare system. An interaction in the sense of the invention can, e.g., include a treatment that a user receives from a healthcare system, a user operating a healthcare-data-management system 1005 or healthcare system to treat another user/patient, or a user-vitals measurement via any medical device like a blood-pressure monitor as a part of a healthcare system. The healthcare-data-management system can also comprise any communication means for exchanging data with any reinforced-protection data base, healthcare system, external device, and/or computing unit.

External device 1007, 1009 can include external user devices, e.g., tablets, smartphones, or computers. Further external devices can include all sorts of wearable electronics, e.g., smartwatches, to monitor user habits and/or user health in real time. A user can be any person who uses or interacts with a healthcare system including patients or healthcare professionals such as nurses and doctors. A user can also be an entity such as a healthcare provider, an insurance company, an external investor, or a medical-technology company.

The reinforced-protection data base 1000 is configured to store requirement data and interaction data, wherein the interaction data and requirement data can be exchanged with the reinforced-protection data base 1000. Interaction data include all sorts of data related to a user interaction with a healthcare system. Requirement data include all sorts of user procedures wherein the performing or triggering the performing of the user procedures depend on a preconfigured conditional expression, e.g., written in a program language, and stored on the reinforced-protection data base 1000. In one example, requirement data and interaction data are exclusively stored on the DL 1001 or the secured server 1003. In another example the requirement data and the interaction data are distributed between both data storages 1001, 1003 or, in general, the requirement data and the interaction data are distributed between more than one reinforced-protection data base. Distributing certain data between the DL 1001 and the secured server 1003 or a plurality of reinforced-protection data bases is technically advantageous since at least a fraction of sensitive data, e.g., as patient data, is non-accessible for public users. Since several reinforced-protection data bases may have different security levels, more sensitive interaction data or requirement data can be separately stored on reinforced-protection data bases having more and/or different means of protection. For example, this can include any additional authentication steps, authentication tokens, or improved encryption and security standards.

The DL 1001 can be any peer-to-peer-type network comprising a plurality of nodes, e.g., servers, computers, or smartphones, having communication means for exchanging data with other nodes of the network. All nodes within the DL comprise storage means, whereby the nodes store a copy of the DL's data records. Storage means can include any of a hard-disc drive (HDD), solid-state drive, or any other data-storage medium. For new data records, e.g., a data record of a digital-asset transfer between two nodes within the DL, the other nodes of the DL verify the new data records and align to a new state of the DL by means of a consensus protocol.

Consensus protocols are required to verify data transfers on a DL and preventing single nodes of a user within the network to alter data records in its favor. Therefore, DLs are usually considered as highly manipulation-resistant and can be one possible example of a reinforced-protection data base. Potential attackers would require unreasonable amounts of resources for altering the DLs data records. A plurality of consensus protocols is known in the state of the art including Proof of Work (PoW), Proof of Stake (PoS), Proof of Space (PoSpace), or Proof of Elapsed Time (PoET). Prominent examples of data systems on the DL can be used for storing data records including Blockchain-type (BC) or Directed-Acyclic-Graph-type (DAG) data systems. In contrast to BC-type data systems, the structure underlying DAG-type data systems have vertices and edges for recording transactions between various counterparties. Due to this tree-type structure, DAGs potentially provide faster, more energy-efficient, and cheaper transactions compared to common BC-type data systems.

DLs can be publicly available or private so that access is only granted to specific user groups. For example, authentication and identification means such as alphanumerical passphrases, identification cards, gestures, or biometrics like fingerprints are required to get access to private DLs. DLs can also be semi-private so that users or user groups can have different permission levels to view, modify, or exchange data records. User groups can be any subgroup of users like patients, doctors, nurses, or external stakeholder like healthcare companies, insurance companies, or private investors having different permission levels on the DL.

Examples for known DLs in the state of the art are Bitcoin and Ethereum DLs using PoW as the consensus protocol of choice. Bitcoin and Ethereum DLs are frequently used for securely trading virtual assets like crypto currencies or NFTs.

The reinforced-protection data base 1000 further comprises a secured server 1003. A secured server can be a server, computing unit, or private network system with restricted availability and/or access for different user groups. For example, accessing the secured server via a computer is only be possible within a specific local-area networks (LAN) or virtual-private networks (VPN). Access to the secured server can be limited via identification and authentication technologies, which can include alphanumeric passphrases, authentication tokens like ID cards, gestures, or biometrics like fingerprints.

The secured server 1003 can store sensitive data records, which shall only be shared with specific nodes of the DL 1001. Examples for such sensitive records can include sensitive patient data, payment data, or other data types, which shall not be stored on a publicly accessible DLs. The secured server can also be optional if, e.g., a private DL is integrated within the DL 1001. Therefore, the reinforced-protection data base 1000 may only comprise a DL 1001 without the secured server 1003, or vice versa, e.g., to reduce the required amount of hardware resources. In such a case, accessing sensitive data on the private DL requires authentication and identification of the user as it is the case for the secured server 1003.

For enhancing data safety on the reinforced-protection data base 1000, in one aspect, multi-factor authentication (MFA) can be used for accessing data records and/or modifying data records on the reinforced-protection data base 1000, i.e., on the DL 1001 and/or the secure server 1003. MFA can comprise a combination of entering a passphrase on a computer and an additional external device such as a user smartphone or a transaction-number (TAN) generator. For example, in response of a log-in attempt of a user with a computer, an alphanumeric code is sent to the user's smartphone, wherein the alphanumeric code is required to get access to the reinforced-protection data base 1000 with the computer.

Data records can also be stored as an information-reduced data object on the reinforced-protection data base 1000. Instead of storing sensitive patient data as a plain-text data object or as an encrypted plain-text data object, an information-reduced data object is generated from the plain-text data object. For example, a hash-type data object or an anonymized data object is stored on the reinforced-protection data base 1000. Hash functions such as Secure Hash Algorithm (SHA) take input values, e.g., sensitive patient data such as patient name, age, or sex, and map the sensitive data onto an alphanumeric and/or symbolic hashed value. Starting from the hashed value, it is impossible to extract the input value, i.e., initial plain-text data object. However, with the knowledge of the initial plain-text data object and the corresponding hash function the hashed value can be reproduced. Sensitive plain-text data objects can securely be stored on the secured server 1003 or any other server external to the reinforced-protection data base 1000.

Data records can also be encrypted and divided in data chunks, wherein the data chunks are distributed on more than a single data base such as a DL or a secured server. One single data chunk, i.e., a fraction of the originally encrypted data record, cannot be used to extract information of the original data record. The recovering of the originally stored information requires all data chunks. Such technologies are known in the art, e.g., see WO 2021 083861.

**Figure 1B** provides an illustration of the method according to the invention. The interaction data IDat 1100 comprises at least some data of a user interaction with a healthcare system, e.g., health data, omics data, or treatment data. The interaction data can be recorded via any data acquisition unit from any external device 1007, 1009, any healthcare system, or any healthcare-data-management system 1005 and stored at least partly on the reinforced-protection data base 1000 as shown in Fig. 1A. The healthcare-data-management system may also comprise any subsystem like healthcare systems, and/or external devices comprising data acquisition units, wherein the subsystems may also be configured to generate interaction data via a respective data acquisition unit.

From the interaction data IDat 1100, at least a fraction of the stored interaction data is extracted for generating 1101 an activity gauge 1102. An activity gauge can be any sort of measure, e.g., a single number or a plurality of numbers, wherein the measure quantifies a user's activity based on the interaction data IDat 1100 as recorded within the system as shown in Fig. 1A. Generating the activity gauge 1102 from the interaction data IDat 1100 can include a mathematical algorithm taking the recorded interaction data IDat 1100 as an input and map the interaction data on said measure using a predefined algorithm. For example, one possible algorithm may add, depending on the type of interaction data, a number of activity points to an activity account of a user, wherein the number of activity points would be the activity gauge of the respective user. Therefore, the user's interaction data would be transformed into a quantitative measure.

The generation 1101 of the activity gauge 1102 from the interaction data IDat 1100 can be performed on any sort of computing unit, wherein the computing unit can be located either on the reinforced-protection data base or any other sort of external computing device having communication means for exchanging data with the reinforced-protection data base, the healthcare-data-management system and/or any other healthcare system. Preferably, the activity gauge is a function of time A(t) 1102 or varies over time. In such case, the real-time activity gauge is a numerical representation of a user's current activity or the activity within an arbitrary time period. With that, the activity gauge 1102 can further be used to detect critical health conditions or unhealthy user behaviors just in time. For example, an emergency notification may be sent to a healthcare provider and/or the user if the activity gauge undercuts a critical activity-gauge threshold or remains within a critical activity-gauge interval or domain for a predefined/critical time period.

The interaction data 1100 can comprise a plurality of different data categories. For example, interaction data 1100 can comprise logging data, health data, omics data, healthcare-system data, adherence data, and/or treatment data. In general, interaction data 1100 can include any data record associated with a user of a user group interacting with a healthcare system. For instance, interaction data can also comprise interactions of healthcare professionals such as nurses or doctors with a healthcare system to monitor their performance with respective performance metrics for assessing, e.g., the quality of care.

Logging data can comprise all sorts of data records associated with user activities on online platforms or health applications, accessed via a user's external device, e.g., a smartphone or computer. In one example, the number of logins on an online platform for users is counted, or how often a user requests information from a health application. A health application can be a computer program which supports, patients, e.g., to live healthier lives by providing lifestyle recommendation like cooking recipes or workout advices. Logging data can also comprise data records of verifiable patient participation at an examination, treatment, or health teaching. Logging data can also comprise activity data of healthcare professional interacting with a healthcare system, e.g., treating or examining a patient with the healthcare system. Keeping track of logging data provides frequent data records so that a user's activity can be monitored within comparably short time scales.

Health data can comprise all sorts of data records associated with a user's health, such as patient vitals, e.g., blood pressure, heart rate, data of blood screenings, and/or data regarding pre-existing and present diseases as well as treatment plans, or examination data. Health data can also comprise personal information of a user such as name, age, or sex.

Omics data can comprise all sorts of data records associated with genomics, proteomics, metabolomics, metagenomics and transcriptomics of a user. For example, omics data can include epigenomics data of patients or relatives, e.g., for root-cause investigations regarding chronic diseases such as chronic kidney disease.

Healthcare-system data can comprise data records regarding the set-up or the maintenance of a healthcare system. Set-up or maintenance data can comprise any data related to the interaction of a user and a healthcare system aside from usual treatments, e.g., data records verifying a correct device set-up. Healthcare-system data can also comprise data concerning the technical status, e.g., data records regarding changed or repaired healthcare-system components or conducted functionality tests.

Adherence data can comprise data, which verify the correct application and dosing, i.e., as prescribed in quantity, quality, frequency, etc., of drugs, medical agents, or medical liquids. For example, various technologies for monitoring patient adherence are known in the art such as drug-ingestion tracking based on images or videos of a patient captured via a user device.

Treatment data comprise all sorts of data aggregated from users during a medical treatment. For example, treatment data can include data such as medical-device-configuration data, patient-vitals data, or data related to treatment events. For example, medical-device-configuration data can comprise treatment pre-settings according to a treatment prescription such as a volume of dialysate used during a dialysis treatment. Patient-vitals data can include all recorded/measured/deduced patient vitals before, during, or after a treatment such as blood pressure, body temperature, or pulse rate. Data related to treatment events can be events which occurred during a treatment session such as treatment interruptions, e.g., due to device errors, warnings, or medical incidents.

In general, the various types of interaction data 1100 allow to monitor deviations from usual user behavior or healthcare-system behavior. Therefore, interaction data 1000 or the activity gauge 1102 as generated from the interaction data can be used, e.g., to anticipate a risk for adverse health events for a patient, like strokes, heart attacks, or acute renal diseases. In one aspect, a decrease or altered pattern of the activity gauge 1102 indicates an adverse health event for the respective user, wherein in response of determining that an adverse health event is expected to occur, an appropriate preconfigured user procedure is performed or performed to be triggered. A preconfigured user procedure can be any user procedure which can only be modified by authorized user groups. Authorized user groups such as healthcare professionals or healthcare companies can upload said preconfigured user procedures to the requirement data stored on the reinforced-protection data base. A preconfigured user procedure can comprise first-aid suggestions or further information being sent to a patient, e.g., in response of determining a low activity gauge, so that the adverse health event is prevented. For example, it is detected that the activity gauge falls below a critical threshold because of missing/insufficient provided interaction data. Due to preconfigured requirement data, a user procedure may then be triggered, wherein the user procedure can comprise a message sent to the respective patient/user and/or the patient's healthcare provider wherein the message may include an activity request, e.g., for the patient/user to respond to the respective message or to log in on a patient web portal, and/or the respective healthcare provider to contact the patient due to an expected emergency or inferred hazardous situation for the patient.

The reinforced-protection data base 1000 of Fig. 1A further stores requirement data 1104. Requirement data 1104 comprise requirements, e.g., conditional expressions C preferably in a program language for performing or triggering a performing of a user procedure U. In one example, a requirement is a threshold value for the activity gauge 1102 below or above which a user procedure U is performed or the performing of the user procedure U is triggered. A requirement can also be an interval of the activity gauge 1102 inside or outside said interval a user procedure U is performed. A user procedure U can be any sort of operation on a set of data, e.g., a transfer of data between one or more users. For example, transferred data can comprise virtual assets like non-fungible tokens (NFTs) or notifications transferred to users, wherein said data transfer is triggered in case that an associated requirement is fulfilled, e.g., the activity gauge falls below one or a plurality of activity-gauge thresholds Ai.

The requirement data 1104 can be created or modified by authorized users or user groups having access to the reinforced-protection data base of Fig. 1A 1000, e.g., via an external device. Modifying requirement data 1104 can include that a user defines that a user procedure U is performed, or the performing is triggered, if the user's activity gauge 1102 undercuts/exceeds one or more critical values, e.g., threshold values. For example, such critical values can be associated with a user inactiveness, e.g., a patient terminates a healthcare program due to an organ transplant or due to death. Allowing users to add and modify requirement data 1104 on the reinforced-protection data base allows them to create immutable legacy-type data records. Immutable data records, e.g., based on distributed-ledger technology, including the seamless performing of preconfigured user procedures provides the unique technical advantage to create both trusted and secure digital legacies. Such records technically simplify post-mortem transfers of digital assets so that data losses and/or losses of valuable assets are prevented. As the data transaction to pre-determined heirs is guaranteed due to distributed-ledger technology, only authorized individuals, i.e., usually the user, have control of the plurality of the preconfigured data transactions of a user procedure U.

In one example, a patient-monitoring device connected to a user/patient is in communication with the healthcare system, the healthcare-data-management system and/or any reinforced-protection data base. A patient-monitoring device can comprise any of a smartwatch, wrist band, or a brain chip for providing real-time interaction data. For instance, the patient-monitoring device may continuously monitor patient vitals, e.g., heart rate, blood pressure, or brain waves, and transmit the acquired interaction data to the reinforced-protection data base. The advantage of real-time monitoring is that deviation of the user's health can be detected immediately so that first-aid or life-saving medication can be provided to the patent in due time.

Moreover, the patient-monitoring device can also detect the death of a person in real time. With that, respective user procedures comprised within the requirement data can be triggered shortly after the death incident. Due to the short time period between a user incident, e.g., the user's death, and the triggering of a user procedure, possible fraud can be prevented since attackers trying to steal the user's digital assets have a much smaller time frame to exploit the user's decease.

From the stored requirement data 1104 and the generated activity gauge 1102, a computing unit monitors 1103 if at least one of the stored requirements within the requirement data 1104 is fulfilled. Upon determining that at least one requirement within the requirement data 1104 is fulfilled, i.e., the conditions C are met, the associated user procedure U is performed, or the performing is triggered by the computing unit. The computing unit can be part of the reinforced-protection data base, e.g., a node of the DL or the secured server, of an external device, of a healthcare-data-management system or of a healthcare system, wherein the external device, the healthcare-data-management system, or the healthcare system is configured to exchange data with the reinforced-protection data base.

In one aspect, the requirement data 1104 and/or the respective user procedures U can be configured as a smart contract on a DL. More advanced DLs, e.g., the Ethereum blockchain, allow the implementation of smart contracts. A smart contract, in a more generalized meaning, can also comprise any type of executable code on the reinforced-protection data base wherein the executable code is triggered and/or performed to be triggered if a predefined condition is met. In this sense, a DL is not necessarily required to employ smart contracts. For example, if no DL is available, a secured server may also send, receive, store, and/or trigger or perform the triggering of any smart contract. Smart contracts can perform a preconfigured directive if a set of conditions is fulfilled. For example, a smart contract can comprise an arbitrary piece of code in a program language, e.g., a conditional data transfer between two or more parties based on an activity gauge, which can only be triggered if the conditions regarding a specific state of the activity gauge is fulfilled. For data records, which are not stored on the DL, but are required for performing the smart contract, blockchain oracles are known in the art and can be used to extract missing data records on the DL from external data sources such as external servers, external devices, or external sensors.

In another aspect of the present invention, based on the performed user procedure U as part of the requirement data 1104, triggering data is generated and stored on the reinforced-protection data base. Triggering data comprise information regarding the performing of the user procedure U. For example, triggering data can include status data comprising information whether a user procedure U, e.g., a data transfer between two or more users, was performed and whether such execution was successful, i.e., without errors. Triggering data can also comprise information about the users taking part in the performed user procedure or about the type of user procedure that was performed, e.g., a health advice was sent to a smartphone of a user as the user's activity gauge fall below some threshold limit.

Triggering data can further be encrypted and/or stored as an information-reduced data object on the reinforced-protection data base. In one example, triggering data of sensitive data are fractionally stored on the DL and the secured server so that extracting the information of the respective triggering data requires all data fractions from the DL and the secured server.

In another example, a hashed value is generated from the triggering data and uploaded on the reinforced-protection data base. The plaintext version of the triggering data remains on the secured server, a user's device, e.g., a smartphone, or any other data storage. The hashed value works as a receipt or a checksum for users to proof that the respective user procedure associated with the triggering data was performed.

The illustrated technical features of Fig. 1A and Fig. 1B underlying the invention provide secure and reliable means to generate and perform data transfers. Due to the reinforced-protection data base. e.g., based on DL technology, users of the system are assured that preconfigured data transfers are preformed if the respective conditions are met. From the user's activity gauge generated from the provided interaction data critical conditions of patients, can early be detected and appropriate countermeasures can be performed. The technical problem of providing forgery-proof, patient-consent-management data platforms in compliance with data-protection regulations is solved by the technical features according to Fig 1A and 1B. Moreover, the DL technology further allows user-activity-dependent data transfers where patient take full control of their data.

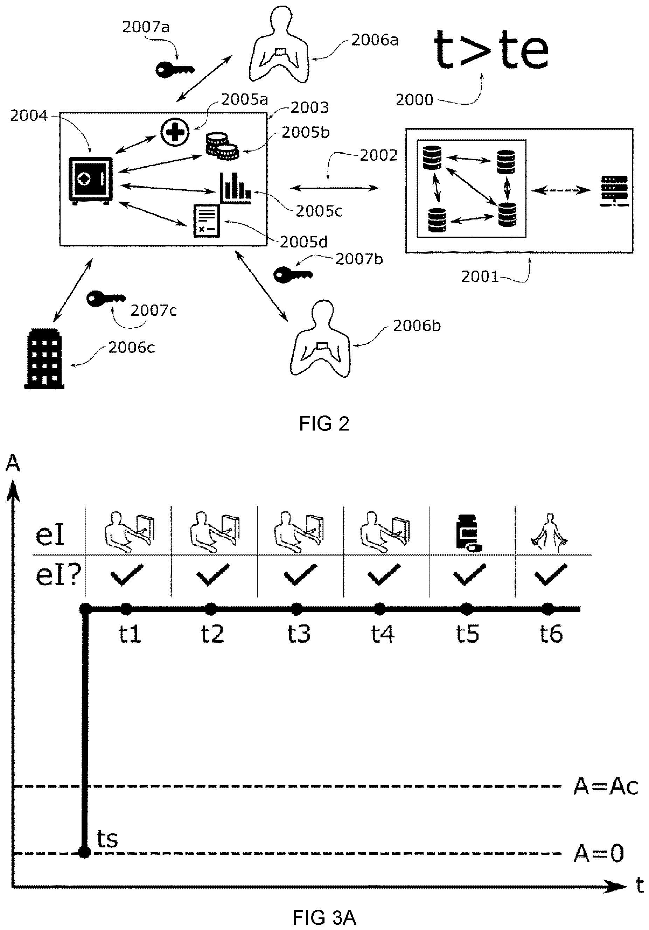

**Figure 2** illustrates an exemplary user procedure 2003 depending on the activity gauge 1102 of Fig. 1B. A computing unit determines that at least some requirements for performing the user procedure 2003 of previously stored requirement data are fulfilled, e.g., a user's generated activity gauge fails to exceed a predefined threshold limit after a critical time t>te 2000. From the requirement data retrieved 2002 from the reinforced-protection data base 2001, the user procedure 2003 is selected, and the triggering is performed by the computing unit. The user procedure 2003 includes a plurality of data transfers associated with digital assets 2005a, 2005b, 2005c, 2005d, wherein the digital assets are stored on a safe data storage 2004. The safe data storage can be any part of the reinforced-protection data base 2001 or any external data-storage device. In one example, a server stores the digital assets the server can be accessed from the information stored on the reinforced-protection data base 2001. Such information can, e.g., comprise encrypted hash keys granting access to the digital assets 2005a, 2005b, 2005c, 2005d.

Digital assets 2005a, 2005b, 2005c, 2005d can be any type of valuable data. In Fig. 2, the digital assets include patient data 2005a, digital tokens 2005b, user statics 2005c, and digital contracts 2005d. Patient data 2005a can comprise any patient-related data such as interaction data. Digital tokens 2005b can comprise fungible and/or non-fungible tokens having any kind of monetary value. User statistics 2005c can include any type of behavioral user data, whereby the behavioral user data may not be part of the patient data 2005a, e.g., data records regarding the user's consumer behavior or the user's personal preferences or believes. Digital contracts 2005b can be conditional obligation or user procedure, wherein the conditional obligations are performed or come into action in response of the user procedure 2003. A digital contract 2005 can be a smart contract, so that further data transfers or owner/user right transfers are performed, or their triggering is performed.

In response of performing the user procedure 2003, the digital assets can be distributed to a plurality of user/parties 2006a, 2006b, 2006c. The parties can be natural persons 2006a, 2006b, e.g., the rightful heirs of a deceased patient, or any corporate body such as a healthcare company 2006c or external investor. The different parties receive digital keys 2007a, 2007b, 2007c for accessing the digital assets on the safe data storage 2004. Depending on the user procedure 2003, in one aspect, the ownership or the digital assets or the digital-asset rights are either fully or partly transferred to the plurality of parties. For example, each party can receive a specific key, which only grants limited access to the set of data. The extent of the right transfer can be part of the user procedure 2003 and controlled, e.g., by regulation stored on the reinforced-protection data base 2001. The modification/change of the ownership of the set of digital assets can be securely recorded 2002 on a blockchain-type data structure on the DL of the reinforced-protection data base 2001.

In one aspect, the step of performing or triggering the performing of a user procedure generates triggering data, wherein triggering data and/or log data thereof are stored on a reinforced-protection data base 2001. Triggering data can be any data regarding the accomplished or not-accomplished user procedure 2003. For example, triggering data can include information about the transferred digital assets, information regarding the participating parties, or triggering-meta data. Triggering-meta data can include all data related to the actual user procedure, e.g., type of the user procedure, time, or transfer costs.

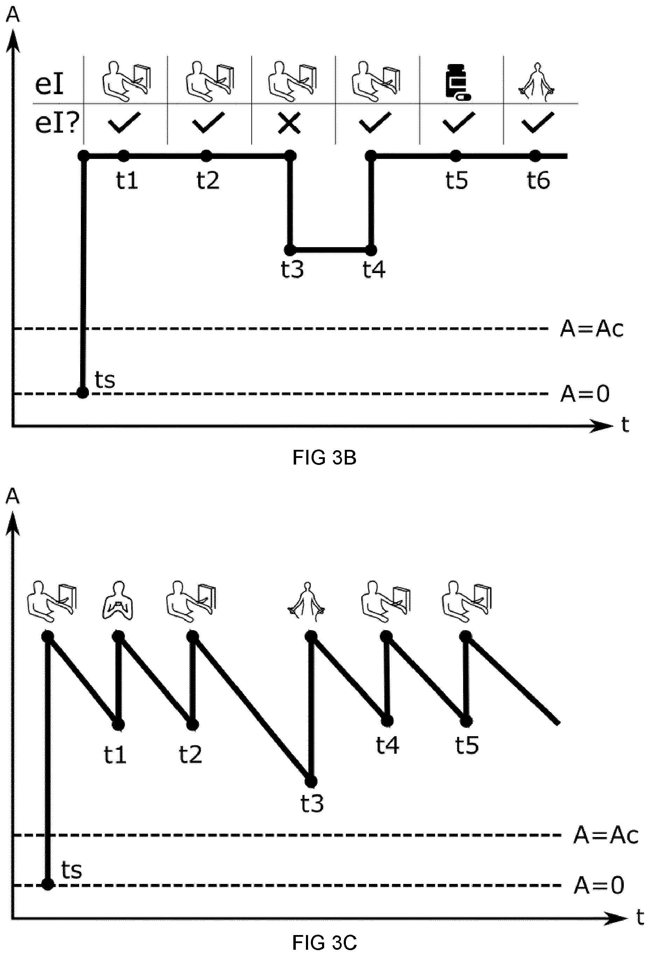

The **Figures 3A** and **3B** show an implementation of the activity gauge 1102 of Fig. 1B in case of an adherent patient (Fig. 3A) and a non-adherent patient (Fig. 3B). In this example, the activity gauge A is a numeric value as a function of time t. Fig. 3A and Fig. 3B further show a value of the activity gauge associated with no activity A=0 as well as a threshold value of the activity gauge A=Ac wherein a user procedure is performed or performed to be triggered in case that the activity gauge undercuts the threshold value of the activity gauge Ac. Each of the Fig. 3A and Fig. 3B illustrate a table, wherein the first row of each table el includes expected interaction data at a given point in time (t1 - t6) and wherein the second row of the matrix el? includes a status, wherein the status determines whether the expected interaction data el was provided at the given point in time and received by the reinforced-protection data base, i.e., whether a user indeed performed a scheduled user interaction.

Fig. 3A illustrates an exemplary activity gauge of a perfect adherent user, e.g., a patient. At a starting point in time ts the user starts tracking the user's activity, e.g., by creating a user account for the respective system. From the provided interaction data, a finite activity gauge is determined by means of a mathematical algorithm. At the points in time t1 - t4, treatments, e.g., dialysis treatments, are scheduled. The user perfectly adheres to the associated prescriptions and receives at each of the time points t1 - t4 the user's treatment. Due to the perfect user adherence, the determined activity-gauge curve based on the provided interaction data, i.e., the expected treatment data, remains on a constant value. At the time point t5, the user is required to take drugs, e.g., renal drugs. The user verifies the intake of the drugs, e.g., by filming the drug intake or by providing corresponding vitals indicating the compliant user behavior. At the point in time t6, the user provides health data, e.g., a body-composition measurement based on bioimpedance spectroscopy. As the user adheres to the required prescriptions by providing the required interaction data at the points in time t5 and t6, the activity gauge remains on the constant value as of the previous time points t1 - t4.

Fig. 3B illustrates an exemplary activity gauge of a non-perfectly adherent user. Comparable to the perfectly adherent patient as illustrated in Fig. 3A, the user starts to track the user's activity, e.g., by creating a user account for the respective system at a starting point in time ts. From the provided interaction data, a finite activity gauge is generated by means of a mathematical algorithm. At the points in time t1 - t2, treatments, e.g., dialysis treatments, are scheduled. The user (Fig. 3A) adheres to the associated prescriptions and receives at each of the time points t1 - t2 the user's treatment. Due to the user's adherence, the generated activity-gauge curve based on the provided interaction data, i.e., the expected treatment data, remains on a constant value. At the point in time t3, the user misses to adhere to the user's prescribed treatment. As no interaction data is provided at t3, the newly generated activity gauge at t3 is lower compared to the generated activity gauge at the point in time t2. Since the user missed the treatment for the first time, the activity gauge remains above the threshold Ac so that no user procedure is performed/triggered. At the point in time t4, the user again adheres to the prescribed treatment. Therefore, interaction data is provided from the treatment at t4 so that the generated activity gauge is reinstated to its value at the point in time t2. As the user further adheres to the required prescriptions by providing the required interaction data at the points in time t5 and t6, the activity gauge remains on the constant value as of the time points t1 - t4.

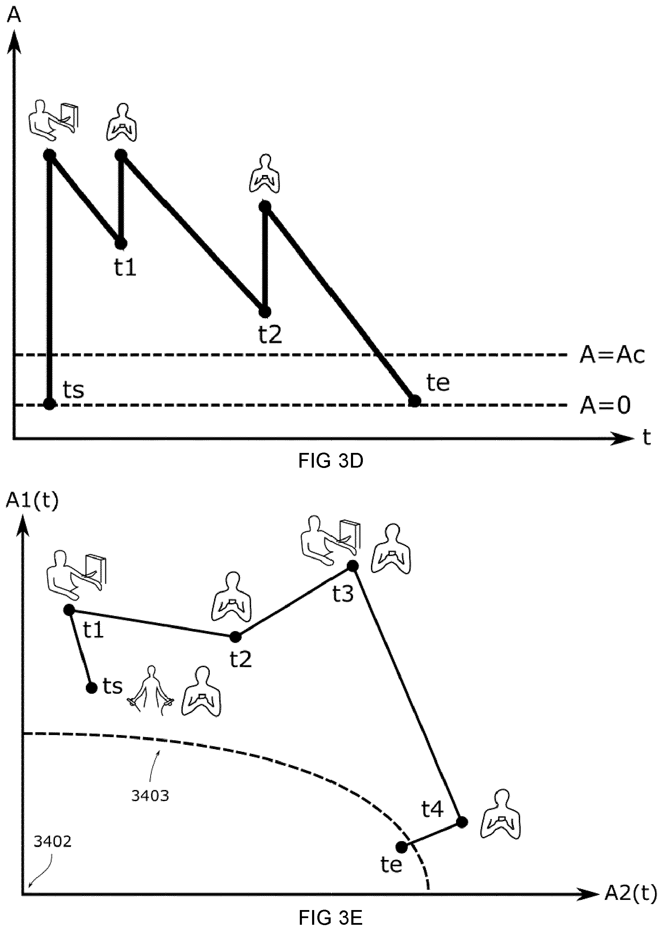

The **Figures 3C** and **3D** show an implementation of the activity gauge 1102 of Fig. 1B in case of a mostly adherent patient (Fig. 3C) and a mostly non-adherent patient (Fig. 3D) whereby the activity gauge includes a discount factor. A discount factor can be any type of activity gauge when added to a first activity gauge yields a second activity gauge wherein the second activity gauge is associated with a reduced or no activity of a user compared to the first activity gauge. In one example, the discount factor comprises a negative number of activity-gauge units in which the user's activity gauge is measured. The negative number of activity-gauge units is added to a first number of activity-gauge units if a user does not adhere to a prescribed treatment, i.e., the expected interaction data of the respective treatment are not received by the reinforced-protection data base. With that, a second number of activity-gauge units is generated wherein the second number of activity-gauge units is associated with a lower user activity compared to the first number of activity-gauge units.

The discount factor can further be a function of time and/or a function of the expected but absent interaction data. For instance, the first activity gauge can be altered towards a second activity gauge associated with a lower activity if no interaction data is received by the reinforced-protection data base within a predefined period of time, e.g., a first activity-gauge value is reduced by a fixed or variable activity-gauge value towards a second activity-gauge value associated with a lower user activity, each second/hour/day where no interaction data is received by the reinforced-protection data base any interaction data. The activity gauge can also be reduced in response that an expected interaction-data record is not received by the reinforced-protection data base, e.g., a patient is non-adherent to a prescribed treatment.

In the example of Figs. 3C and 3D, the activity gauge A is a numeric value as a function of time t and includes a discount factor. Fig. 3C and Fig. 3D show a value of the activity gauge associated with no activity A=0 as well as a threshold value of the activity gauge A=Ac wherein a user procedure is triggered in case that the activity gauge undercuts the threshold value of the activity gauge Ac. At each of the points in time ti, a user interaction of the user is recorded and received by the reinforced-protection data base. From the received user interaction, an activity gauge is generated using a mathematical algorithm.

Fig. 3C illustrates an exemplary activity gauge of a mostly adherent user. The user starts to track the user's activity at a starting point in time ts when first interaction data, e.g., treatment data, are recorded and transmitted to the reinforced-protection data base. As no interaction data is received between the points in time ts and t1, the activity gauge continuously decreases towards activity-gauge values associated with a lower user activity. At the point in time t1, the user generates logging data so that the value of the activity gauge is again increased towards higher activity-gauge values associated with a higher user activity. The pattern repeats itself for the depicted following points in time t2 - t5. For the time period between the points in time t2 and t3, the user does not provide intermediary interaction data so that the activity gauge decreases towards values close to the threshold Ac. As the user provides interaction data, i.e., health data, at t3 a positive activity-gauge value is added to the current activity-gauge value yielding an activity gauge close to the peak of the curve at the point in time t2.

Fig. 3D illustrates an exemplary activity gauge of a mostly non-adherent user. The user starts to track the user's activity at a starting point in time ts when first interaction data, e.g., treatment data, are recorded and transmitted to the reinforced-protection data base. As no interaction data is received between the points in time ts and t1, the activity gauge continuously decreases towards activity-gauge values associated with a lower user activity. At the point in time t1, the user provides logging data so that the generated activity gauge is shifted towards activity-gauge values associated with a higher user activity. For the time period between the points in time t1 and t2, the user does not provide intermediary interaction data so that the activity gauge decreases towards values close to the threshold Ac. At t2, the user provides logging data, shifting the activity gauge upwards towards higher activity-gauge values. Between the points in time t2 and te no more interaction data of the user is received. The activity-gauge curve undercuts the threshold limit Ac so that a predefined user procedure is performed, e.g., a reminder is sent to the user to adhere to the user's treatment prescription. As no further interaction is provided, the activity gauge falls to A=0.

In the examples of Fig. 3C and 3D, the disclosed discount factor is a constant value with dimension activity-gauge per unit time, whereby the constant value is subtracted from the current activity-gauge value in each time step. In general, the discount factor is not limited to a constant value and can, e.g., also include more involved functional mappings such as exponential-type or power-law-type scaling behaviors. For example, an exponential mapping can sanction long-term inactivity, more strictly, compared to a linear scaling of the discount factor so that users are more motivated to stay compliant to their prescription. The discount factor can also depend on the absolute time since the starting time ts and vary over the time. This might be advantageous for better incentivizing long-term users with a variable discount factor.

In one aspect, the discount factor relies on an artificial-intelligence model. Historical user behavior can be employed to train a neural network to optimize a reward function, e.g., to maximize the activity gauge. A combination of a variation of discount factors, threshold limits and associated user procedures can be used to generate a strategy for motivating a user to stay active and adhere to the user's prescriptions or tasks. Mathematical models which can be employed to optimize such strategies can be based on Markov-Decision Processes (MDPs) in the framework of reinforcement learning as it is known in the state of the art.

In one aspect, the step of generating an activity gauge relies on a mathematical algorithm. A mathematical algorithm can be any type of functional connection or transformation between the acquired input, i.e., the interaction data, and generated output, i.e., the activity gauge. The activity gauge reflecting a user's activity based on the user's provided interaction data can be any sort of an alphanumeric value, symbol, color, or other type of visual, acoustic, or haptic feedback on a user device, e.g., a smartphone, a healthcare system and/or a data base.

In another aspect, the activity gauge includes a mathematical metric. In general, a mathematical metric allows measuring a distance within an abstract mathematical space, e.g., Euclidean metrics are examples for metrics frequently used in Euclidean space. The mathematical metric can be employed to determine a distance between two activity-gauge values. For example, a distance between a predefined threshold value below/above which a user procedure is performed, and the current activity gauge of a user as generated from the user's interaction data. An activity gauge can be a numerical object, e.g., a single number, for which a plurality of mathematical metrics like the absolute value are known. For higher-dimensional representations of activity gauges, e.g., a matrix including a plurality of numbers, a plurality of matrix norms or operator norms such as p-norms:

$$\|x\|_p \equiv \left( \sum_{i=1}^{n} |x_i|^p \right)^{1/p}$$

are known in the state of the art.

For non-numerical activity gauges, a mathematical metric can be defined. In one example, an associated numerical representation of a non-numerical activity gauge, e.g., a symbol or a color, on a computer system is used, e.g., a hexadecimal number associated with the symbol or color, to define a respective mathematical metric. For example, information-technology standards such as Unicode provide alphanumeric representations of symbols so that mathematical metrics can be deduced based on such alphanumeric representations. A type of mathematical metric can also be defined based on the non-numerical hierarchy of activity-gauge values. For example, in case of a binary system, e.g., having an "active" and "inactive" state, a numerical object such as a "1" is associated with the "active" state where the "inactive" is associated with a "0". For the so-deduced numerical objects, mathematical metrics known in the art can be applied.

In another aspect, the mathematical algorithm includes a credit system whereby each user interaction is associated with a specific type and/or number of credits, whereby a credit score is associated with a user and whereby there is a one-to-one correspondence between the credit score and an activity gauge.

For example, in case that interaction data is generated, based on the data category and the modality of the interaction data, a specific amount of credits is added to a user's credit score stored on the reinforced-protection data base. Modalities can include all types of information necessary for assessing the value or of the corresponding interaction data to adapt the credit weightings for the credit system. For example, such modalities can include information regarding the percentage and timeliness of exercised treatments or information regarding adherence habits to a medication according to a prescribed dosage. Specific data categories, e.g., treatment data, can result in a higher number of credits compared to other data categories, e.g., logging data.

In another aspect, specific interaction data associated with different data categories can contribute to different types of credit scores, i.e., different sorts of credits can only be associated with specific data categories of interaction data. Missing interaction data, e.g., due to patient non-adherence to a prescribed treatment, can lead to a decrease of the credit score by subtracting a predefined number of credits from a user's credit score, i.e., the activity gauge of a user decreases towards activity gauges associated with a lower user activity. The evaluation of an activity, i.e., the provided interaction data, to an amount of credit can depend, e.g., on the user and time, and is generally not fixed.

In another aspect, the mathematical algorithm comprises a stochastic algorithm. For example, based on new interaction data of a user, i.e., interaction data generated within a predefined short time scale, and history-interaction data, i.e., stored interaction data from a user's past or from other user's interaction data, an inference model is trained to generate an activity gauge wherein the activity gauge reflects the probability of a user for being active in a future time. For example, such a state can be defined such that a specific user has a high probability for further generating interaction data within a predefined future time or not.

Such inference models may be any kind of a supervised and/or unsupervised machine-learning model. For example, data such as first interaction data, of previous user behavior may be employed to train a machine-learning model, e.g., to predict first user procedures and/or any first generated activity gauge at later points in time. Such machine-learning model may comprise a neural network based on a tree-based-type architecture, long-short-term-memory-type architecture, or any other type of neural-network architecture suited from the state of the art for predicting any user procedure and/or activity gauge at later points in time. Then, the trained machine-learning model can be employed for predicting most probable second user interactions which are expected to be triggered and/or any second activity gauge at later points in time from a user's second interaction data.

In another example, an unsupervised and/or self-learning machine-learning model based, e.g., on a neural network employing reinforcement learning based on a Markov-Decision Process, is employed for predicting a user procedure and/or any activity gauge at a later point in time from incoming interaction data. An unsupervised and/or self-learning machine-learning model has the advantage that the prediction accuracy and/or the model's decision quality can continuously be optimized. For example, the model may select at any point in time from a number of actions from a user, e.g., requesting specific interaction data like specific patient vitals. The machine-learning model may adjust the type and/or frequency of actions depending on the outcome, i.e., the triggered user procedures, and/or the user's activity gauge at later points time, of the set of performed actions so that a reward function is maximized/optimized. One possible reward function may be the activity gauge of the user, wherein the activity gauge shall be maximized. Therefore, the machine-learning model may develop a strategy for maximizing a user's activity gauge so that, e.g., patient remain active and adhere to their prescribed treatments yielding measurable longer and heathier patient lives.

In another aspect, the step of generating an activity gauge associated with a user at least partly based on the interaction data includes a multi-dimensional activity gauge. A multi-dimensional activity gauge allows the tracking of a user's activity in cases in which a single activity gauge is insufficient to properly reflect a user's activity. For example, a user can have more than a single role, e.g., a patient and a clinician role, so that a multi-dimensional activity gauge allows to simultaneously track all user roles. Multi-dimensional activity gauges for different data categories of interaction data are also technically advantageous to prevent users to misuse the activity tracking, e.g., users must comply to life-saving treatments and cannot artificially elevate the user's activity gauge by only providing logging data.

In another aspect, a multi-dimensional activity gauge can be mapped on a single activity gauge to generate a compound activity gauge. In one example, a weighted sum or map such as an arithmetic mean or median or a norm, e.g., the absolute value, of the multi-dimensional activity gauge is used to generate a compound activity gauge from the plurality of activity gauges.

Figure 3E illustrates a two-dimensional activity gauge as an example for a multi-dimensional activity gauge. The two-dimensional activity gauge comprises a first activity-gauge dimension A1, e.g., associated with a first data category such as treatment and health data, and a second activity-gauge dimension A2, e.g., associated with a second data category such as logging data, wherein both activity-gauge dimensions depend on the time t. The Fig. 3E includes a point associated with no user activity 3402 (A1 =A2=0) and a dotted threshold curve 3403 (Ac) as a function of A1 and A2, whereby inside the area as delimited by the axis A1, the axis A2, and the curve Ac, a user procedure is performed. At a first point in time ts, a user provides health data and logging data whereby the activity gauge acquires a finite value as generated based on the interaction data. At t1, the user performs a treatment and generates treatment data so that the new activity gauge is shifted towards higher A1 values. Since no logging data is provided at t1, the user's activity gauge is shifted towards lower A2 values due to a discount factor continuously shifting the activity gauge towards 3402. At t2, the user provides logging data so that the user's generated activity gauge is shifted towards larger A2 values. Due to the discount factor, the user's activity gauge is shifted towards smaller A1 values. Both treatment data and logging data are provided at t3 so that the generated activity gauge is increased in A1 and A2 dimensions. Since the user does not comply with the user's treatment prescription, the user's activity gauge strongly decreases towards small A1 values at t4. Since logging data is provided, the activity gauge is shifted to higher A2 values at t4. No further interaction data of any data category is provided so that the user's activity gauge undercuts the threshold curve 3403 (Ac) at the point in time te performing a user procedure or triggering the performing of a user procedure.

The advantage of multi-dimensional activity gauges relies in the elevated accuracy to measure a user's activity with respect to the provided type/data category of interaction data. The plurality of activity-gauge dimensions allows to generate/create multi-dimensional requirement data so that user-customized requirements, i.e., threshold curves/hyper planes, can be created. With that, the user's behavior can be tracked in more detail and compliant user behavior, e.g., adherence to prescriptions, can be fostered.

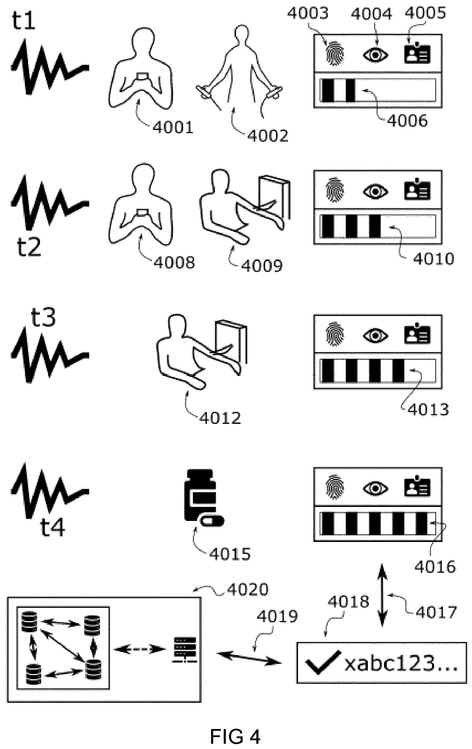

**Figure 4** shows an illustration of a quantity gauge according to an embodiment of the invention. At a first point in time t1 a user generates interaction data, e.g., logging data 4001 and health data 4002, and optionally authenticates via biometrics, e.g., fingerprints/palm prints 4003 or retina scan 4004, or any authentication token 4005, e.g., an identification card or passphrase. In this example, the quantity gauge is illustrated as a progression bar 4006 wherein the progression bar is filled if interaction data is generated within a predefined time limit. The intermediately generated quantity gauge 4006 can either be stored locally on a user's external device, a healthcare system, a healthcare-data-management system, and/or be stored on an external server, e.g., a reinforced-protection data base.

A quantity gauge can be any activity gauge where the activity of a user is measured based on the frequency of generated interaction data. For example, no further information than the generation of interaction data is correlated to generate a quantity gauge. In another example, a multi-dimensional quantity gauge is generated, e.g., for each data category of the interaction data a separate quantity gauge is generated.

At the point in time t2 the user continues generating interaction data 4008, 4009 within the predefined time limit so that the progress bar 4010 associated with the quantity gauge progresses. The user continues generating interaction data 4012, 4015 within a predefined time limit at the time points t3 and t4 so that the progress bar 4013, 4014 is further filled. In case that sufficient interaction data is generated within the time limit, e.g., the number of generated interaction data exceeds one more threshold values within the predefined time, a corresponding information from the quantity gauge is generated 4017. The quantity-gauge data 4018 generated from the quantity gauge includes a positively evaluated quantity of interaction data within the respective time limits so that the quantity gauge and/or the generated quantity-gauge data 4018 are transferred 4019 and stored on the reinforced-protection data base 4020.

In one aspect, insufficient interaction data is generated within a time limit, e.g., due to user inactivity, whereby the quantity-gauge data 4018 generated from the quantity gauge includes a negatively evaluated quantity of interaction data within the time limit. In this case, the quantity gauge and/or the generated quantity-gauge data 4018 are not or only partly transferred and stored on the reinforced-protection data base 4020.

In one aspect, the quantity gauge is part of an activity gauge wherein the quantity gauge is encoded in the activity-gauge value. In general, the activity gauge can be a numerical object, e.g., a number or a plurality of numbers, wherein the frequency of the generated interaction data, i.e., the quantity gauge, is comprised in the one or more activity-gauge values. With that the one or more activity-gauge value also reflects the quantity gauge. For example, a positively evaluated quantity of user interactions increases the other activity gauges, e.g., a bonus activity-gauge value is added to the other activity gauges.

In another aspect, the quantity gauge is a separate type of activity gauge of a multi-dimensional activity gauge. In such case, the activity gauge is split in two or more dimensions, e.g., in two or more numerical objects such as a tuple of numbers, wherein the two or more dimensions of the activity gauge independently reflect the quantity gauge and any other type of generated activity gauge, e.g., based on the data category of the generated interaction data.

In another aspect, the quantity gauge is part of an activity gauge wherein the quantity gauge is encoded in the activity-gauge value and the quantity gauge is a separate type of activity gauge of a multi-dimensional activity gauge. In this case, the activity gauge comprises a multi-dimensional activity gauge wherein a first activity gauge comprises a combination of the quantity gauge and any other activity gauge generated from the type/quality of provided interaction data and wherein a second type of activity gauge of the multi-dimensional activity gauge only includes the quantity gauge. The first and the second activity gauge can be numerical object and/or non-numerical objects. For example, the first activity gauge and/or the second activity gauge can be a symbol or a color, e.g., traffic-light colors.

In another aspect, a healthcare system for verifiably controlling data transfers may comprise a data acquisition unit configured to acquire interaction data of a user wherein interaction data include data of an interaction between a user and the healthcare system. The healthcare system may include communication means configured to exchange interaction data with a first reinforced-protection data base, wherein the healthcare system is configured to transmit interaction data to the first reinforced-protection data base. The healthcare system is further configured to generate an activity gauge at least partly based on the interaction data and wherein the first or a second reinforced-protection data base is configured to store requirement data including information related to the performing of a user procedure associated with a data transfer depending on the activity gauge. The healthcare system and/or the first or second reinforced-protection data base are further configured to communicate with a computing unit and wherein the computing unit is configured to perform and/or to trigger the performing of the user procedure depending on the activity gauge.

**Claims**

1. A computer-implemented method for verifiably controlling data transfers, comprising the steps:

   - Retrieving interaction data associated with a user from a first reinforced-protection data base; wherein interaction data include data of an interaction between a user and a healthcare system
   - Generating an activity gauge associated with the user at least partly based on the interaction data;
   - Retrieving requirement data from the first and/or a second reinforced-protection data base,
   wherein requirement data include information related to the performing of a user procedure associated with a data transfer depending on the activity gauge
   - Performing and/or triggering the performing of the user procedure depending on the activity gauge

2. Method according to claim 1, wherein the first and/or second reinforced-protection data base comprise a distributed ledger.

3. Method according to anyone of the preceding claims, wherein the step of performing and/or triggering the performing of a user procedure generates triggering data, wherein the triggering data and/or log data thereof are stored on the first and/or second reinforced-protection data base.

4. Method according to claim 2 or 3, wherein the distributed ledger comprises a blockchain-type and/or a directed-acyclic-graph-type data system.

5. Method according to anyone of the preceding claims, wherein the step of performing and/or triggering the performing of the user procedure is implemented as a smart contract on the reinforced-protection data base.

6. Method according to anyone of the preceding claims, wherein the interaction data are derived by sensing and/or acquiring one or more of logging data, health data, omics data, healthcare-system data, adherence data, and/or treatment data.

7. Method according to anyone of the preceding claims, wherein the activity gauge includes a discount factor.

8. Method according to anyone of the preceding claims, wherein the step of generating an activity gauge at least partly based on the interaction data comprises generating a multi-dimensional activity gauge.

9. Method according to anyone of the preceding claims, wherein the step of generating an activity gauge at least partly based on the interaction data comprises generating a quantity gauge.

10. Method according to anyone of the preceding claims, wherein the step of generating an activity gauge at least partly based on the interaction data comprises generating a separate quantity gauge for each data category of the interaction data.

11. Method according to the claim 9 or 10, wherein the step of performing and/or triggering the performing of a user procedure is conditional on characteristics of the quantity gauge.

12. Method according to anyone of the preceding claims, wherein the reinforced-protection data base is configured to read or receive and store interaction data from an external device.

13. A computer program comprising instructions which, when the program is executed by a computing device, cause the computing device to carry out a method according to anyone of the claims 1 to 12.

14. A computer-readable medium having stored thereon the computer program of claim 13.

15. A healthcare-data-management system for verifiably controlling data transfers, comprising:

   - A data acquisition unit configured to acquire interaction data including data of an interaction between a user and a healthcare system;
   - communication means configured to exchange the interaction data with a first reinforced-protection data base and/or with a second reinforced-protection data base;

   wherein the healthcare-data-management system is configured to:

   - generate an activity gauge at least partly based on the interaction data;
   - retrieve requirement data from the first and/or second reinforced-protection data base, wherein the requirement data include information related to the performing of a user procedure associated with a data transfer depending on the activity gauge;

- perform and/or trigger the performing of the user procedure depending on the activity gauge.

FIG 1A

FIG 1B

FIG 2

FIG 3A

FIG 3B

FIG 3C

FIG 3D

FIG 3E

FIG 4

Europäisches
Patentamt

European
Patent Office

Office européen
des brevets

# EUROPEAN SEARCH REPORT

Application Number

EP 22 18 3873

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MEKOUAR LOUBNA ET AL: "A survey on blockchain-based Recommender Systems: Integration architecture and taxonomy", COMPUTER COMMUNICATIONS, ELSEVIER SCIENCE PUBLISHERS BV, AMSTERDAM, NL, vol. 187, 7 February 2022 (2022-02-07), pages 1-19, XP087014368, ISSN: 0140-3664, DOI: 10.1016/J.COMCOM.2022.01.020 [retrieved on 2022-02-07] * 6 A modular architecture for blockchain-based recommender systems * ----- | 1-6,8-15 | INV. G06F21/62 G06F21/64 |
| A | US 2019/354693 A1 (YOON WOONG A [US] ET AL) 21 November 2019 (2019-11-21) * paragraph [0032] - paragraph [0039] * * paragraph [0044] - paragraph [0050] * ----- | 1-15 | |
| A | US 2020/389309 A1 (RICOTTA FRANK [US] ET AL) 10 December 2020 (2020-12-10) * paragraph [0090] - paragraph [0110] * ----- | 1-15 | |

TECHNICAL FIELDS
SEARCHED (IPC)

G06F

The present search report has been drawn up for all claims

| Place of search | Date of completion of the search | Examiner |
|---|---|---|
| Munich | 6 December 2022 | Chabot, Pedro |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...........................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 18 3873

This annex lists the patent family members relating to the patent documents cited in  the above-mentioned European search report.
The members are as contained in the European Patent Office EDP file on
The European Patent Office is in no way liable for these particulars which are merely given for the purpose of information.

06-12-2022

| Patent document cited in search report | Publication date | Patent family member(s) | Publication date |
|---|---|---|---|
| US 2019354693 A1 | 21-11-2019 | NONE | |
| US 2020389309 A1 | 10-12-2020 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021083861 A